# EUROPÄISCHE PATENTSCHRIFT

(11) **EP 1 266 924 B1**
(45) Veröffentlichungstag und Bekanntmachung des Hinweises auf die Patenterteilung: **28.06.2006**
(21) Anmeldenummer: 02012337.8
(22) Anmeldetag: 05.06.2002
(51) Int. Cl.: C08G 64/14, C08G 63/20, C08G 63/64, C07C 39/15

(54) **Polycarbonate und Polyestercarbonate mit verzweigten Endgruppen**
Polycarbonates and polyestercarbonates having branched endgroups
Polycarbonates et polyestercarbonates ayant des groupes terminaux rétifiés

(30) Priorität: 13.06.2001 DE 10128705
(43) Veröffentlichungstag der Anmeldung: 18.12.2002
(73) Patentinhaber: Bayer MaterialScience AG, 51368 Leverkusen (DE)
(72) Erfinder: Heuer, Helmut-Werner, Dr., 47829 Krefeld (DE); Wehrmann, Rolf, Dr., 47800 Krefeld (DE); Meyer, Alexander, Dr., 47800 Krefeld (DE); Pielartzik, Harald, Dr., 47800 Krefeld (DE); Bruder, Friedrich Karl, Dr., 47802 Krefeld (DE); Paulusse, Jos M. J., Dr., 5615 GA Eindhoven (NL)

(56) Entgegenhaltungen:
- DE-A- 4 141 718
- US-A- 3 166 606
- US-A- 4 929 709
- US-A- 5 043 403
- MILLER T M ET AL: "SYNTHESIS OF FOUR GENERATIONS OF MONODISPERSE ARYL ESTER DENDRIMERS BASED ON 1,3,5-BENZENETRICARBOXYLIC ACID" MACROMOLECULES, AMERICAN CHEMICAL SOCIETY. EASTON, US, Bd. 25, Nr. 12, 8. Juni 1992 (1992-06-08), Seiten 3143-3148, XP000269456 ISSN: 0024-9297
- ATSUSHI MORIKAWA ET AL: "CONVERGENT SYNTHESIS OF STARBURST POLY(ETHER KETONE) DENDRONS" MACROMOLECULES, AMERICAN CHEMICAL SOCIETY. EASTON, US, Bd. 26, Nr. 24, 22. November 1993 (1993-11-22), Seiten 6324-6329, XP000413598 ISSN: 0024-9297

## Beschreibung

Die Erfindung betrifft die Verwendung von Phenolen mit verzweigter Struktur als Endgruppen in Polycarbonaten und Polyestercarbonaten sowie Polycarbonate und Polyestercarbonate mit verzweigten Endgruppen, Formkörper und Extrudate aus diesen Polymeren, Verfahren zur Herstellung der Polymere sowie der Formkörper und Extrudate, die verzweigten Phenole selbst und deren Herstellung.

Zur Herstellung von Polycarbonaten werden häufig monofunktionelle Endgruppen auf Phenolbasis wie z.B. Plienol, 4-Alkylphenole und 4-Cumylphenol eingesetzt (Kunststoff-Handbuch 3; L. Bottenbruch, Hanser, München 1992, S. 127; EP 0 353 594 A1).

Es ist nicht bekannt, dass sich diese herkömmlich eingesetzten Endgruppen positiv auf das Fließverhalten und/oder die Nullviskosität und damit positiv auf die Verarbeitungseigenschaften der entsprechenden Polycarbonate auswirken.

Die Herstellung von solchen Polycarbonaten mit verzweigten Endgruppen ist grundsätzlich bekannt und bspw. in EP-A 0 794 209 und JP-A 06 256 499 beschrieben.

Aus den US-B 3 166 606 und 3 173 891 ist beispielweise *p*-Phenylphenol als Kettenabbrecher für Polycarbonate bekannt. Aus US-B 4 330 663 sind Polyestercarbonate bekannt, in welchen die 4-Benzylbenzoesäure als Kettenabbrecher eingesetzt wird (Spalte 6, Zeile 18).

WO-A 00/50 488 beschreibt die Verwendung von Di-*tert.*-alkylphenol als Kettenabbrecher.

Aus der japanischen Offenlegungsschrift 57 13 31 49 sind Polycarbonate bekannt, die mit Phenylpropylphenol, Alkylphenolen oder Naphthol als Endgruppen modifiziert sind.

Aus JP-A 06 25 64 99 sind Polycarbonate mit Endgruppen der Strukturen 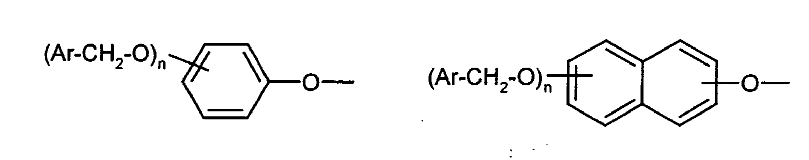 bekannt.

Gemäß DE-A 38 03 939 werden Kettenabbrecher der Formel 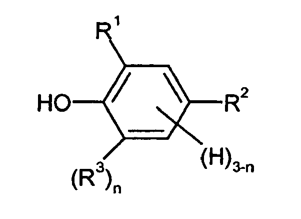 eingesetzt,
worin
R¹, R², R³ gleich oder verschieden sind und C₂-C₁₂-Alkyl oder C₈-C₂₀-Aralkyl sind, wobei mindestens einer der Reste R¹ oder R² ein C₈-C₂₀-Aralkyrest ist, und worin "n" einen Wert zwischen 0.5 und 1 hat.

Diese bekannten Polycarbonate, Polyestercarbonate und Polyester besitzen aber häufig den Nachteil einer hohen Schmelzviskosität.

Ausgehend vom Stand der Technik stellt sich somit die Aufgabe, Polycarbonate und Polyestercarbonate , bzw. geeignete Phenole als Endgruppen zu Verfügung zu stellen, welche den Nachteil einer hohen Schmelzviskosität vermeiden.

Überraschenderweise wurde nun gefunden, dass diese Aufgabe durch den Einsatz von Endgruppen mit spezieller verzweigter, insbesondere dendrimerartiger, Struktur, gelöst wird. Diese Endgruppen beeinflussen die Schmelzviskosität positiv, d.h. das entsprechende Polycarbonat zeigt bei vergleichbarer Molekulargewichtsverteilung eine niedrigere Schmelzviskosität und ist damit besser verarbeitbar.

Phenolische Endgruppen mit dendrimerartiger Struktur auf Basis von ester- oder ether-verbrückten Arylsystemen sind bislang nicht bekannt.

Gegenstand der vorliegenden Erfindung sind daher Polycarbonate und Polyestercarbonate enthaltend verzweigte, insbesondere dendrimerartige Endgruppen, die Verwendung solcher Polycarbonate und spezielle, zur Verwendung in den erfindungsgemäßen Polycarbonaten geeignete phenolische Endgruppen bzw. die zugrunde liegenden Phenole.

Gegenstand der vorliegenden Erfindung sind daher auch die Verwendung der Phenole gemäß Formel (2) zur Herstellung endgruppenmodifizierter Polymere - ausgenommen Bis-(C₁-C₁₈-alkylphenyl)-4-hydroxyisophthalate (DE-A 19 62 229) unsubstituiertes Diphenylhydroxyisophthalat und Bis-[(diphenyloxycarbonyl)-phenyl]-hydroxyisophthalat (T. M. Miller, E. W. Kwock, T. X. Neenan, *Macromolecules* **1992,** *25,* 3143-3148), unsubstituiertes Diphenyloxyphenol (DE-A 16 44 922), 3,5-Dibenzoylphenol (Dischendorfer et al., *Monatsh. Chem.* **1935,** *66,* 255), 4-Methyl-2,6-dibenzoylphenol (S. K. Gupta et al., *Transition Met. Chem*. **1997,** *22,* 372-374), 2-Metyl-4,6-dibenzoylphenol (K. C. Amin et al. *J. Indian Chem. Soc.* **1960,** *37*, 469-472) und 3,5Bis-(4-hydroxybenzoyl)phenol (A. Morikawa, M. Kakimoto, Y. Imai *Macromolecules* **1993,** *26*, 6325) - selbst, sowie deren Darstellung.

Die Phenole der Formel (2) sind wie folgt definiert: 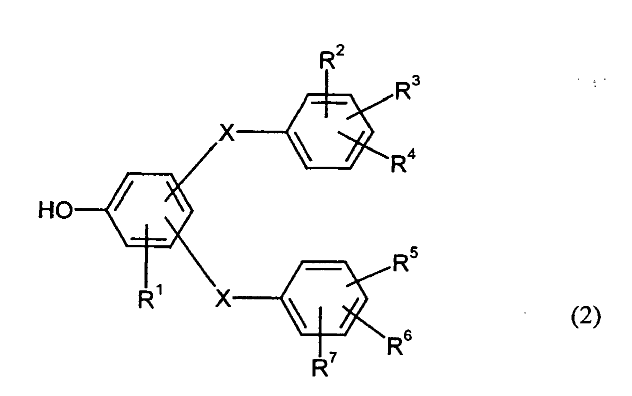 worin
- R¹, R², R⁷: unabhängig voneinander für H, lineares oder verzweigtes C₁-C₁₈ Alkyl-, Cl, oder Br, bevorzugt für H oder lineares oder verzweigtes C₁-C₁₂ Alkyl-, besonders bevorzugt für H oder C₁-C₈ Alkyl-, und ganz besonders bevorzugt alle für den gleichen Rest, insbesondere H stehen,
- X: kann eine Einfachbindung oder ein divalentes Radikal wie -CO₂-, -O-, -CH₂ oder -CO- sein, bevorzugt sind dabei Ester-verbrückte (2a), Ether-verbrückte (2b) und Carbonyl-verbrückte (2c) Arylbausteine,
- R³ - R⁶: sind unabhängig voneinander H, lineare oder verzweigte C₁-C₁₈ Alkyl-, cyclisches C₅-C₁₈ Alkyl-, Phenyl-, Phenyloxy-, Phenylcarboxy-, Benzyl-, Benzyloxy-, Naphthyl- oder Naphthyloxy-, Naphthylcarboxyreste, bevorzugt sind H, lineare oder verzweigte C₁-C₁₂ Alkyl-, cyclisches C₅-C₁₂ Alkyl-, Phenyloxy-, Benzyloxy- oder Naphthyloxyreste und besonders bevorzugt jeweils der gleiche Rest insbesondere H, linear oder verzweigtes C₁-C₁₂ Alkyl-, cyclisches C₅-C₁₂ Alkyl-, Phenyloxy- oder Benzyloxyrest.

Besonders bevorzugt sind jeweils unabhängig voneinander die Phenole, welche den Formeln 2a bis 2c entsprechen: 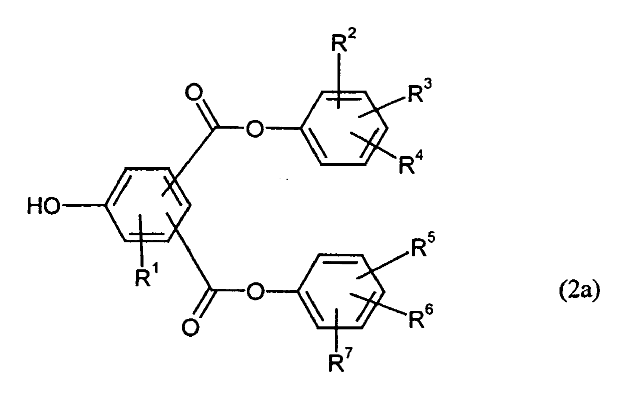 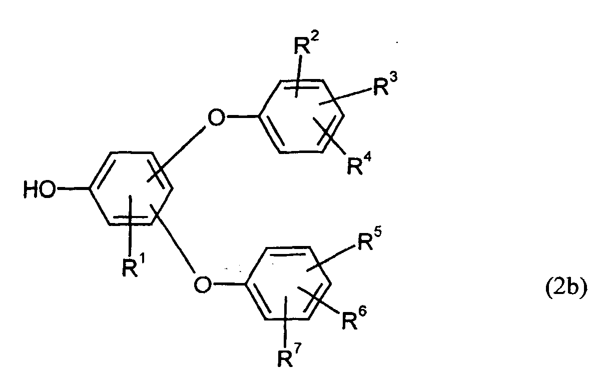 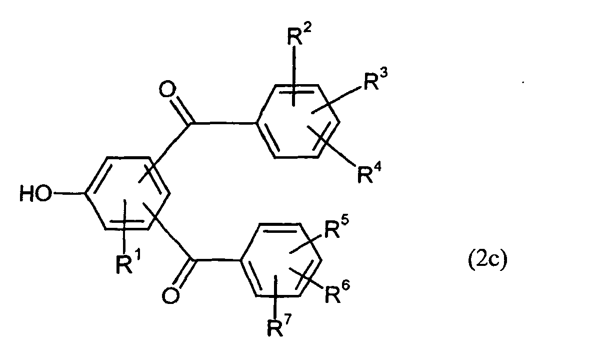 wobei in 2a bis 2c die Reste R¹ bis R⁷ die oben genannten Bedeutungen haben.

Geeignete Endgruppen zur Modifikation von Polycarbonaten und Polyestercarbonaten sind durch Formel (3a) dargestellt:
Besonders geeignet sind die Endgruppen der Formel (3a) 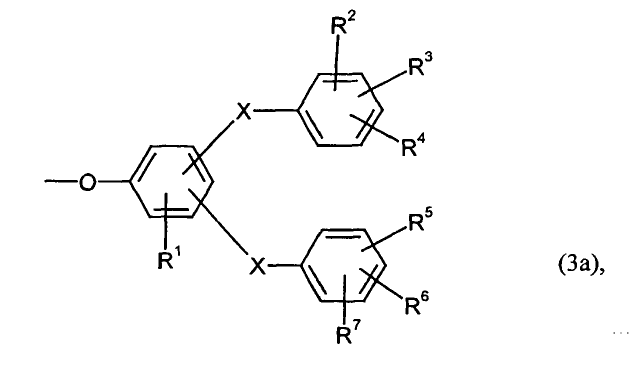
worin

X, und R¹-R⁷ die oben genannten Bedeutungen haben.

Unabhängig voneinander ganz besonders geeignet sind die den Phenolen der Formeln (2a) bis (2c) entsprechenden Endgruppen.

Bevorzugt, besonders bevorzugt, ganz besonders bevorzugt oder insbesondere bevorzugt etc. sind Verbindungen, welche die unter bevorzugt, besonders bevorzugt, ganz besonders bevorzugt oder insbesondere bevorzugt etc. genannten Substituenten tragen.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Gegenstand der vorliegenden Erfindung sind somit auch thermoplastische Polycarbonate und thermoplastische Polyestercarbonate mit den Phenolen der Formeln (2) entsprechenden Endgruppen.

Beispiele für Phenole sind 3,5-Diphenyloxyphenol, 3,5-Bis-(*p-tert*.-butylphenyloxy)-phenol, 3,5-Bis-(*p-n*-butylphenyloxy)-phenol, 1,3-Diphenyl-5-hydroxyisophthalat, 1,3-Bis-(*p-tert*.-butylphenyl)-5-hydroxyisophthalat, 1,3-Bis-(*p-iso-*octylphenyl)-5-hydroxyisophthalat, 1,3-Bis-(3,5-di-tert.-butylphenyl)-5-hydroxyisophthalat, 1,3-Bis-[*p*-(2-phenylprop-2-yl)-phenyl]-5-hydroxyisophthalat, 1,3-Dicyclooctyl-5-hydroxyisophthalat, 1,3-Dicyclododecyl-5-hydroxyisophthalat und 1,3-Bis-[3,5-(diphenyloxycarbonyl)-phenyl]-5-hydroxyisophthalat.

Die erfindungsgemäß einzusetzenden Monophenole sind nach literaturbekannten Verfahren herstellbar (T. M. Miller E. W. Kwock, T. X. Neenan, *Macromolecules* **1992,** *25*, 3143-3148, A. Morikawa, M. Kakimoto, Y. Imai, *Macromolecules* **1993,** *26,* 6324-6329). Von denen unter den Ausführungsbeispielen beschriebenen Phenolen sind 1,3-Diphenyl-5-hydroxyisophthalat und 1,3-Bis-[3,5-(diphenyloxycarbonyl)-phenyl]-5-hydroxyisophthalat (T. M. Miller E. W. Kwock, T. X. Neenan, *Macromolecules* **1992,** *25*, 3143-3148) und 3,5-Diphenyloxyphenol (DE-A 16 44 922) bekannt. Von Phenolen der Formel (2a) sind Bis-(C₁-C₁₈-alkylphenyl)-4-hydroxyisophthalate aus DE -A 19 62 229 bekannt.

Außer den Phenolen der Formel (2) können noch andere Phenole in Mengen bis zu 50 Mol-% bezogen auf die jeweilige Gesamtmenge an Kettenabbrecher zur Herstellung der Polycarbonate, Polyestercarbonate und Polyester mitverwendet werden.

Gegenstand der vorliegenden Erfindung ist somit auch die Verwendung der Phenole der Formel (1) gegebenenfalls in Kombination mit anderen Phenolen als Kettenabbrecher zur Herstellung von aromatischen Polycarbonaten, aromatischen Polyestercarbonaten und aromatischen Polyestern, wobei die anderen Phenole in Mengen bis zu 50 Mol-% vorzugsweise bis 25 Mol-% bezogen auf die jeweilige Gesamtmolmenge an Kettenabbrecher eingesetzt werden.

Gegenstand der vorliegenden Erfindung sind somit auch thermoplastische Polycarbonate und thermoplastische Polyestercarbonate der Formel (4) 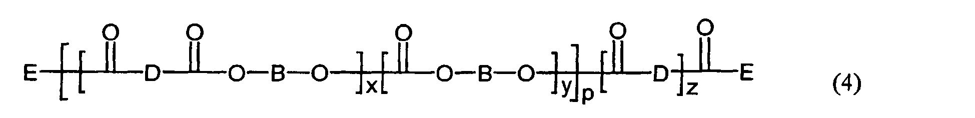 worin 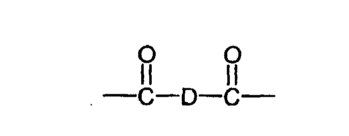 der Rest einer aromatischen Dicarbonsäure ist, -O-B-O- ein Bisphenolatrest ist, "p" eine ganze Zahl zwischen 25 und 700 ist, "x und y" Bruchzahlen aus der Reihe O/p, 1/p, 2/p bis p/p sind, wobei x + y = 1 ist und "z" = Null oder 1 ist und mindestens 50 Mol-% der Reste E in (4) den Phenolatresten entsprechend den Phenolen der Formel (2) entsprechen und maximal 50 Mol-% der Reste E in (4) einem anderen Phenolatrest als dem entsprechend den Phenolen der Formel (1) entsprechen.

Gemäß DE-A 2 119 799 erfolgt die Herstellung von Polycarbonaten unter Beteiligung phenolischer Endgruppen, nach dem Phasengrenzflächenverfahren wie auch dem Verfahren in homogener Phase.

Zur Herstellung von Polycarbonaten nach dem Phasengrenzflächenverfahren sei beispielhaft auf H. Schnell, Chemistry and Physics of Polycarbonates, Polymer Reviews, Vol. 9, Interscience Publishers, New York 1964 und auf Polymer Reviews, Vol. 10, "Condensation Polymers by Interfacial and Solution Methods", Paul W. Morgan, Interscience Publishers, New York 1965, Kap. VIII, S. 325 verwiesen.

Daneben ist die Herstellung der erfindungsgemäßen Polycarbonate auch nach dem bekannten Polycarbonatverfahren in der Schmelze, dem sogenannten Schmelzumesterungsverfahren, möglich, das z.B. in WO-A 01/05 866 oder in WO-A 01/05 867 beschrieben ist. Daneben werden Umesterungsverfahren (Acetatverfahren und Phenylesterverfahren) beispielsweise in den US-B 34 94 885, 43 86 186, 46 61 580, 46 80 371 und 46 80 372, in den EP-A 26 120, 26 121, 26 684, 28 030, 39 845, 39 845, 91 602, 97 970, 79 075, 14 68 87, 15 61 03, 23 49 13 und 24 03 01 sowie in den DE-A 14 95 626 und 22 32 977 beschrieben.

Diarylcarbonate im Sinne vorliegender Erfindung sind solche Kohlensäurediester der Formel (5) 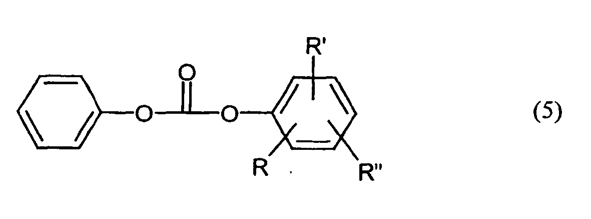 und Formel (6), 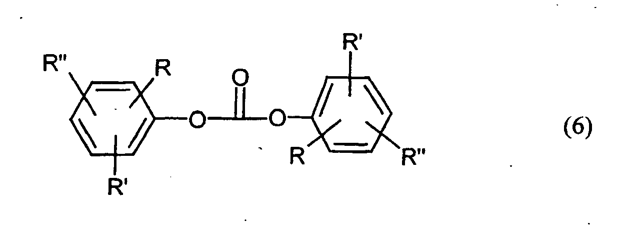 wobei R, R' und R" unabhängig voneinander H, gegebenenfalls verzweigte C₁-C₃₄₋Alkyl/Cycloalkyl, C₇-C₃₄-Alkaryl oder C₆-C₃₄-Aryl bzw. C₆-C₃₄-Aryloxy darstellen können, beispielsweise Diphenylcarbonat, Butylphenyl-phenylcarbonat, Di-butylphenylcarbonat, Isobutylphenyl-phenylcarbonat, Di-isobutylphenylcarbonat, tert.-Butylphenyl-phenylcarbonat, Di-tert-butylphenylcarbonat, n-Pentylphenyl-phenylcarbonat, Di-(n-pentylphenyl)carbonat, n-Hexylphenyl-phenylcarbonat, Di-(n-hexylphenyl)carbonat, Cyclohexylphenylphenylphenolcarbonat, Di-cyclohexylphenylcarbonat, Phenylphenol-phenylcarbonat, Diphenylphenolcarbonat, Isooctylphenyl-phenylcarbonat, Di-isooctylphenylcarbonat, n-Nonylphenyl-phenylcarbonat, Di-(n-nonylphenyl)carbonat, Cumylphenyl-phenylcarbonat, Dicumylphenylcarbonat, Naphthylphenyl-phenylcarbonat, Di-naphthylphenylcarboant, Di-tert-butylphenyl-phenylcarbonat, Di-(di-tert.-Butylphenyl)carbonat, Dicumylphenylcarbonat, Di-(dicumylphenyl)carbonat, 4-Phenoxyphenyl-phenylcarbonat, Di-(4-phenoxyphenyl)-carbonat, 3-Pentadecylphenyl-phenylcarbonat, Di-(3-pentadecylphenyl)carbonat, Tritylphenyl-phenylcarbonat, Di-tritylphenylcarbonat,
bevorzugt
Diphenylcarbonat, tert.-Butylphenyl-phenylcarbonat, Di-tert.-Butylphenylcarbonat, Phenylphenol-phenylcarbonat, Di-phenylphenolcarbonat, Cumylphenyl-phenylcarbonat, Dicumylphenylcarboant,
besonders bevorzugt Diphenylcarbonat.

Diphenole für solche Polycarbonate können beispielsweise Hydrochinon, Resorcin, Dihydroxybiphenyle, Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren kernalkylierte und kernhalogenierte Verbindungen sein und auch α,ω-Bis-(hydroxyphenyl)-polysiloxane.

Bevorzugte Diphenole sind beispielsweise 4,4'-Dihydroxybiphenyl (DOD), 2,2-Bis-(4-hydroxyphenyl)-propan (Bisphenol A), 1,1-Bis-(4-hydroxyphenyl)-3,3,5-trimethylcyclohexan (Bisphenol TMC), 1,1-Bis-(4-hydroxyphenyl)-cyclohexan, 2,4-Bis-(4-hydroxyphenyl)-2-methylbutan, 1,1-Bis-(4-hydroxyphenyl)-1-phenylethan, 1,1-Bis-(4-hydroxyphenyl)-*p*-diisopropylbenzol, 1,3-Bis[2-(4-hydroxyphenyl)-2-propyl]-benzol (Bisphenol M), 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 2,2-Bis-(3,5-dichlor-4-hydroxyphenyl)-propan und 2,2-Bis-(3,5-dibrom-4-hydroxyphenyl)-propan.

Besonders bevorzugte Diphenole sind beispielsweise 2,2-Bis-(4-hydroxyphenyl)-propan (Bisphenol A), 1,3-Bis[2-(4-hydroxyphenyl)-2-propyl]-benzol (Bisphenol M), 2,2-Bis-(3,5-dimethyl-4-hydroxyphenyl)-propan, 1,1-Bis-(4-hydroxyphenyl)-1-phenylethan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Die Diphenole können sowohl allein als auch im Gemisch miteinander verwendet werden; es sind sowohl Homopolycarbonate als auch Copolycarbonate einbezogen. Die Diphenole sind literaturbekannt oder nach literaturbekannten Verfahren herstellbar (siehe z.B. H. J. Buysch et al., Ullmann's Encyclopedia of Industrial Chemistry, VCH, New York 1991, 5. Ed., Vol. 19, p. 348).

Es können auch geringe Mengen, vorzugsweise Mengen zwischen 0,05 und 2,0 Mol-%, bezogen auf die Mole eingesetzter Diphenole, an tri- oder multifunktionellen Verbindungen, insbesondere solchen mit drei oder mehr als drei phenolischen Hydroxygruppen als sogenannte Verzweiger, mitverwandt werden. Einige der verwendbaren Verbindungen mit drei oder mehr als drei phenolischen Hydroxygruppen sind beispielsweise Phloroglucin, 4,6-Dimethyl-2,4,6-tri-(4-hydroxyphenyl)-hept-2-en, 4.6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methylbenzyl)-4-methylphenol, 2-(4-Hydroxyphenyl)-2-(3,4-dihydroxyphenyl)-propan, Hexa-[4-(4-hydroxyphenyl-isopropyl)-phenyl]-orthoterephthalsäureester, Tetra-[4-(4-hydroxyphenyl-isopropyl)-phenoxy]-methan, Tetra-(4-hydroxyphenyl)-methan und 1,4-Bis-(4',4"-dihydroxytriphenyl)-methylbenzol.

Weitere mögliche Verzweiger sind 2,4-Dihydroxybenzoesäure, Trimesinsäure, Cyanurchlorid und 3,3-Bis-(3-methyl-4-hydroxyphenyl)-2-oxo-2,3-dihydroindol.

Die gegebenenfalls mitzuverwendenden 0,05 bis 2 Mol-%, bezogen auf eingesetzte Diphenole, an Verzweigern können entweder mit den Diphenolen selbst und den erfindungsgemäßen Molekulargewichtsreglern in der wässrigen alkalischen Phase vorgelegt werden, oder in einem organischen Lösungsmittel gelöst vor der Phosgenierung zugegeben werden.

Die aromatischen Polycarbonate der vorliegenden Erfindung besitzen Gewichtsmittelmolekulargewichte M_{w} (ermittelt durch Gelpermeationschromatographie und Eichung mit Polystyrolstandard) zwischen 5 000 und 200 000, vorzugsweise zwischen 10 000 und 80 000 und besonders bevorzugt zwischen 15 000 und 40 000.

Die relativen Lösungsviskositäten, liegen entsprechend zwischen 1,10 und 1,60 gemessen in Methylenchlorid (0,5 g Polycarbonat in 100 ml Methylenchlorid bei 23°C).

Erfindungsgemäße Polyestercarbonate sind solche, die aus mindestens einem Diphenol, aus mindestens einer aromatischen Dicarbonsäure und aus Kohlensäure aufgebaut sind.

Geeignete aromatische Dicarbonsäuren sind beispielsweise Orthophthalsäure, Terephthalsäure, Isophthalsäure, *tert*.-Butylisophthalsäure, 3,3'-Diphenyldicarbonsäure, 4,4'-Diphenyletherdicarbonsäure, 4,4'-Diphenylsulfondicarbonsäure, 3,4'-Benzophenondicarbonsäure, 2,2-Bis-(4-carboxyphenyl)-propan, Trimethyl-3-phenylindan-4,5-dicarbonsäure.

Von den aromatischen Dicarbonsäuren werden besonders bevorzugt die Terephthalsäure und/oder Isophthalsäure eingesetzt.

Geeignete Diphenole sind die vorstehend für die Polycarbonatherstellung genannten. Die Kohlensäure kann entweder via Phosgen oder via Diphenylcarbonat in die Polyestercarbonate eingebaut werden, je nach Wahl des Herstellungsverfahrens, also je nachdem, ob Phasengrenzflächenpolykondensation oder Schmelzumesterung zur Polyestercarbonatherstellung verwendet wird.

Entsprechendes gilt für die aromatischen Dicarbonsäuren; sie werden entweder als aromatische Dicarbonsäuredichloride im Zweiphasengrenzflächenverfahren oder als Dicarbonsäurediester im Schmelzumesterungsverfahren eingesetzt.

Die Herstellung der erfindungsgemäßen Polyestercarbonate erfolgt nach bekannten Herstellungsverfahren, also wie bereits erwähnt beispielsweise nach dem Phasengrenzflächenverfahren oder nach dem Schmelzumesterungsverfahren.

Die erfindungsgemäßen Polyestercarbonate können sowohl linear als auch in bekannter Weise verzweigt sein. Die aromatischen Polyestercarbonate der vorliegenden Erfindung haben mittlere Gewichtsmittelmolekulargewichte M_{w} (ermittelt durch Gelpermeationschromatographie mit Polystyroleichung) vorzugsweise zwischen 10 000 und 250 000.

Das molekulare Verhältnis von Carbonateinheiten zu aromatischen Dicarboxylateinheiten in den erfindungsgemäßen Polyestercarbonaten liegt vorzugsweise bei 95:5 bis 5:95, besonders bevorzugt zwischen 90:10 bis 10:90, insbesondere bevorzugt zwischen 80:20 und 20:80 und ganz besonders bevorzugt zwischen 60:40 und 40:60, idealerweise bei 50:50.

Die einzusetzende Menge an erfindungsgemäßen Monophenolen der Formel (2) zur Herstellung der erfindungsgemäßen Polycarbonate oder Polyestercarbonate liegt zwischen 0,5 Mol-% und 8 Mol-%, vorzugsweise zwischen 2 Mol-% und 6 Mol-% bezogen auf die jeweils eingesetzten Diphenole.

Als weitere Kettenabbrecher sind die üblichen Monophenole wie beispielsweise Phenol, 4-Alkylphenole und 4-Cumylphenol geeignet.

Gegenstand der vorliegenden Erfindung ist somit auch ein Verfahren zur Herstellung der erfindungsgemäßen Polycarbonate, Polyestercarbonate oder Polyester aus Diphenolen, Monophenolen, Kohlensäurederivaten und/oder Dicarbonsäurederivaten nach an sich bekannten Verfahrensbedingungen, das dadurch gekennzeichnet ist, dass man als Kettenabbrecher Monophenole der Formel (1) in Mengen von 0,5 Mol-% bis 8 Mol-%, vorzugsweise von 2 Mol-% bis 6 Mol-%, bezogen jeweils auf Mole Diphenole, einsetzt, wobei davon bis zu 50 Mol-%, vorzugsweise bis zu 25 Mol-%, bezogen jeweils auf die Gesamtmenge an Kettenabbrecher, durch andere Monophenole ersetzt werden können.

Im Falle des Phasengrenzflächenpolykondensationsverfahrens können die Kettenabbrecher der Formel (2) vor, während oder nach der Phosgenierung in Lösung zugesetzt werden. Die für das Lösen der Kettenabbrecher der Formel (2) geeigneten Lösungsmittel sind beispielsweise Methylenchlorid, Chlorbenzol oder Acetonitril sowie Mischungen dieser Lösungsmittel.

Gegenstand der Erfindung sind auch die nach dem erfindungsgemäßen Verfahren erhältlichen Polycarbonate, Polyestercarbonate und Polyester.

Diphenole zur Herstellung der erfindungsgemäßen Polycarbonate und Polyestercarbonate können auch Polymerisate oder Kondensate mit phenolischen Endgruppen sein, so dass erfindungsgemäß auch Polycarbonate bzw. Polyestercarbonate mit Blockstrukturen einbezogen sind.

Die erfindungsgemäßen Polycarbonate und Polyestercarbonate können in bekannter Weise aufgearbeitet und zu beliebigen Formkörpern verarbeitet werden.
Beispielsweise durch Extrusion oder Spritzguss.

Den erfindungsgemäßen Polycarbonaten und Polyestercarbonaten können noch andere aromatische Polycarbonate und/oder andere aromatische Polyestercarbonate und/oder andere aromatische Polyester in bekannter Weise zugemischt werden.

Den erfindungsgemäßen Polycarbonaten und Polyestercarbonaten können noch die für diese Thermoplasten üblichen Additive wie Füllstoffe, UV-Stabilisatoren, Thermostabilisatoren, Antistatika und Pigmente in den üblichen Mengen zugesetzt werden; gegebenenfalls können das Entformungsverhalten, das Fließverhalten, und/oder die Flammwidrigkeit noch durch Zusatz externer Entformungsmittel, Fließmittel, und/oder Flammschutzmittel verbessert werden (z.B. Alkyl- und Arylphosphite, -phosphate, -phosphane, -niedermolekulare Carbonsäureester, Halogenverbindungen, Salze, Kreide, Quarzmehl, Glas- und Kohlenstofffasern, Pigmente und deren Kombination. Solche Verbindungen werden z. B. in WO99/55772, S. 15 - 25, und in "Plastics Additives", R. Gächter und H. Müller, Hanser Publishers 1983, beschrieben).

Die erfindungsgemäßen Polycarbonate und Polyestercarbonate, gegebenenfalls in Abmischung mit anderen Thermoplasten und/oder üblichen Additiven können zu beliebigen Formkörpern/Extrudaten verarbeitet überall dort eingesetzt werden, wo bereits bekannte Polycarbonate Polyestercarbonate und Polyester eingesetzt werden. Aufgrund ihres Eigenschaftsprofils eignen sie sich insbesondere als Substratmaterialien für optische Datenspeicher wie z.B. CD, CD-R, DVD, oder DVD-R, sind aber auch beispielsweise als Folien im Elektrosektor als Formteile im Fahrzeugbau und als Platten für Abdeckungen im Sicherheitsbereich einsetzbar. Weitere mögliche Anwendungen der erfindungsgemäßen Polycarbonate sind:
1. Sicherheitsscheiben, die bekanntlich in vielen Bereichen von Gebäuden, Fahrzeugen und Flugzeugen erforderlich sind, sowie als Schilde von Helmen.
2. Herstellung von Blaskörpern (siehe z.B. US-Patent 2 964 794).
3. Herstellung von lichtdurchlässigen Platten, insbesondere Hohlkammerplatten, beispielsweise zum Abdecken von Gebäuden wie Bahnhöfen, Gewächshäusern und Beleuchtungsanlagen.
4. Zur Herstellung von Ampelgehäusen oder Verkehrsschildern.
5. Zur Herstellung von Schaumstoffen (siehe z.B. DE-A 1 031 507).
6. Zur Herstellung von Fäden und Drähten (siehe z.B. DE-A 1 137 167 und DE-A 1 785 137).
7. Als transluzente Kunststoffe mit einem Gehalt an Glasfasern für lichttechnische Zwecke (siehe z.B. DE-A 1 554 020).
8. Zur Herstellung von Präzisionsspritzgussteilchen, wie z.B. Linsenhalterungen. Hierzu verwendet man Glasfasern, die gegebenenfalls zusätzlich etwa 1 - 10 Gew.-% MoS₂, bezogen auf Gesamtgewicht, enthalten.
9. Zur Herstellung optischer Geräteteile, insbesondere Linsen für Foto- und Filmkameras (siehe z.B. DE-A 2 701 173).
10. Als Lichtübertragungsträger, insbesondere als Lichtleiterkabel (s. z.B. EP-A 0 089 801)
11. Als Elektroisolierstoffe für elektrische Leiter und für Steckergehäuse sowie Steckerverbinder.
12. Als Trägermaterial für organische Fotoleiter.
13. Zur Herstellung von Leuchten, z.B. Scheinwerferlampen, als sogenannte "head-lamps" oder Streulichtscheiben.
14. Für medizinische Anwendungen, z.B. Oxygenatoren, Dialysatoren.
15. Für Lebensmittelanwendungen, wie z.B. Flaschen, Geschirr und Schokoladenformen.
16. Für Anwendungen im Automobilbereich, z.B. in Bereichen, in denen es zu Kontakt zu Kraftstoffen und Schmiermitteln kommt.
17. Für Sportartikel, wie z.B. Slalomstangen.
18. Für Haushaltsartikel, wie z.B. Küchenspülen und Briefkastengehäusen.
19. Für Gehäuse, wie z.B. Elektroverteilerschränke.
20. Herstellung von Folien, insbesondere Skifolien.
21. Herstellung optischer Datenspeicher.
22. Herstellung von Mobiltelefongehäusen mit verbesserter Beständigkeit gegenüber Parfüm, Rasierwasser und Hautschweiß.
23. Network interface devices.
24. Transparente Waschmaschinen - Bullaugen mit verbesserter Beständigkeit gegenüber der Waschlösung.
25. Schutzbrillen, optische Korrekturbrillen.
26. Lampenabdeckungen für Kücheneinrichtungen mit verbesserter Beständigkeit gegenüber Küchendunst, insbesondere Öldämpfen.
27. Verpackungsfolien für Arzneimittel.
28. Chip-Boxen und Chip-Träger.
29. Für sonstige Anwendungen, wie z.B. Stallmasttüren oder Tierkäfige.

Die Formkörper und Extrudate aus den erfindungsgemäßen Polymeren sind ebenfalls Gegenstand dieser Anmeldung.

### Beispiele

### Beispiel 1

### Darstellung von 1,3-Bis-(tert.-butyldimethylsilyl)-5-(tert.-butyldimethylsiloxy)-isophthalat

In einem Kolben mit aufgesetztem Rückflusskühler werden unter Argonatmosphäre 41,9 g (0,23 mol) 5-Hydroxyisophthalsäure und 94,0 g (1,38 mol) Imidazol in 400 ml *N,N-*Dimethylformamid gelöst. Zu dieser Lösung wird tropfenweise eine Lösung von 139,8 g (0,932 mol) tert.-Butyldimethylsilylchlorid in 765 ml *N,N*-Dimethylformamid gegeben. Das Gemisch wird auf 57°C erwärmt. Nach 11,5 Stunden wird die Reaktion abgekühlt und die abgeschiedenen Kristalle isoliert und in *n*-Hexan gelöst. Das restliche *tert.*-Butyldimethylsilylchlorid wird mit 0,5 M NaOH hydrolisiert. Die Lösung wird mit Wasser gewaschen und über Magnesiumsulfat getrocknet. Nach Trocknung im Hochvakuum verbleibt ein weißer Feststoff (100,7 g, 83,4 %).
¹H-NMR (400 MHz, CDCl₃) δ = 8.26 (s, 1H), 7.70 (s, 2H), 1.03 (s, 18H), 0.99 (s, 9H), 0.39 (s, 12H), 0.12 (s, 6H).

### Beispiel 2

### Darstellung von 5-(tert.-Butyldimethylsiloxy)-isophthalsäure

In einem 41 Kolben werden 100,7 g (0,191 mol) 1,3-Bis-(*tert*.-butyldimethylsilyl)-5-(*tert*.-butyldimethylsiloxy)-isophthalat in einem Gemisch aus 400 ml THF, 1 200 ml Eisessig und 400 ml Wasser gelöst. Die Lösung wird für 3 Stunden gerührt. Man verdünnt mit Wasser und kühlt die Lösung auf 0°C ab, woraufhin sich Kristalle abscheiden. Diese werden abfiltriert und im Hochvakuum getrocknet (43,6 g, 77,0 %).
¹H-NMR (400 MHz, CDCl₃) δ = 12.9 (s 2H), 8.22 (s, 1H), 7.52 (s, 2H), 0.97 (s, 9H), 0.22 (s, 6H).

### Beispiel 3

### Darstellung von 5-(tert.-Butyldimethylsiloxy)-isophthalsäuredichlorid

In einem 1 l Kolben mit aufgesetztem Rückflusskühler werden 43,6 g (0,1471 mol) 5-(tert.-Butyldimethylsiloxy)-isophthalsäure unter Argon vorgelegt. Dazu werden 335 ml Thionylchlorid getropft. Die Mischung wird auf 75°C erwärmt. Nach 10,5 Stunden wird das verbleibende Thionylchlorid unter reduziertem Druck abdestilliert. Man erhält ein braunes Öl (7,29 g, 64,1 %).
¹H-NMR (400 MHz, CDCl₃) δ = 8.45, (s, 1H), 7.84 (s, 2H), 1.02 (s, 9H), 0.28 (s, 6H).

### Beispiel 4

### Darstellung von 1,3-Bis-(p-tert.-butylphenyl)-5-(tert.-butyldimethylsiloxy)-isophthalat

In einem 500 ml Kolben werden unter Argon 7,88 g (52,5 mmol) *p-tert*-Butylphenol, 150 ml Dichlormethan, und 45 ml Pyridin vorgelegt. Zu dieser Mischung werden langsam 7,29 g (21,9 mmol) 5-(*tert*.-Butyldimethylsiloxy)-isophthalsäuredichlorid gelöst in 25 ml Dichlormethan getropft. Die Lösung wird für 6 Stunden bei Raumtemperatur gerührt. Die Lösung wird eingeengt und der Rückstand in Dichlormethan aufgenommen. Die Lösung wird mit 200 ml 1 M NaOH, 200 ml 1 M HCI, 400 ml gesättigter NaCl-Lösung und abschließend nochmals mit Wasser gewaschen und über MgSO₄ getrocknet. Nach Entfernen des Lösungsmittels im Vakuum erhält man das Produkt als weißes Pulver (9,2 g, 55,8 %).
¹H-NMR (400 MHz, CDCl₃) δ = 8.44 (s, 1H), 7.79 (s, 2H), 7.35 (d, 4H), 1.23 (s, 18H), 1.18 (s, 9H)

### Beispiel 5

### Darstellung von 1,3-Bis-(p-tert.-butylphenyl)-5-hydroxyisophthalat

In einem 500 ml Kolben mit aufgesetztem Rückflusskühler werden 9,20 g (16,43 mmol) 1,3-Bis-(*p-tert*.-butylphenol)-5-(*tert*.-butyldimethylsiloxy)-isophthalat, 175 ml Aceton und 25 ml 1 M HCl gegeben. Die Lösung wird für 9 Stunden bei 50°C und für 3 Tage bei Raumtemperatur gerührt. Aceton und Wasser werden dann im Vakuum entfernt. Der Rückstand wird in 400 ml Dichlormethan gelöst. Man wäscht mit 400 ml NaCl-Lösung und abschließend mit 400 ml Wasser. Es wird über MgSO₄ getrocknet und entfernt die flüchtigen Bestandteile im Vakuum. Das Rohprodukt wird in 25 ml Toluol aufgenommen und in 500 ml *n*-Hexan ausgefällt. Nach Filtration und Trocknen im Hochvakuum erhält man das Produkt als weißen Feststoff (6,23 g, 85 %).
¹H-NMR (400 MHz, CDCl₃) δ =10.5 (s, 1H), 7.84 (s, 2H), 7.51 (d, 4H), 7.25 (d, 4 H), 1.35 (s, 18H).

### Beispiel 6

### Darstellung von 1,3-Dibenzyl-5-(benzyloxy)-isophthalat

Unter Argon werden in einem Kolben 5,01 g (27,5 mmol) 3-Hydroxyisophthalsäure und 16,59 g (120 mmol) Kaliumcarbonat vorgelegt. Man gibt 80 ml *N,N*-Dimethylformamid hinzu und tropft zu dieser Lösung bei Raumtemperatur unter rühren 16,51 g (96,5 mmol) Benzylbromid. Anschließend wird auf 57°C erwärmt und lässt bei dieser Temperatur noch 48 Stunden rühren. Die Lösung wird dann abgekühlt und auf Eis gegossen. Es wird mit Ethylacetat extrahiert. Die organische Phase wird mehrfach mit gesättigter NaCl-Lösung gewaschen. Man trocknet die organische Phase über Magnesiumsulfat, filtriert und entfernt das Lösungsmittel im Vakuum.

Man erhält einen weißen Feststoff, welcher aus Ethylacetat umkristallisiert wird (5,2 g, 42 %, Fp: 95,7°C).
¹H-NMR (400 MHz, CDCl₃) δ = 8.36 (s, 1H), 7.85 (s, 2H), 7.5-7.3 (m, 15H), 5.37 (s,4H), 5.13 (s, 2H).

### Beispiel 7

### Darstellung von 1-Benzyloxyisophthalsäure

Zu einer Mischung aus 2,81 g (0,05 mol) Kaliumhydroxid, 33 ml THF und 50 ml Methanol werden 4,52 g (0,01 mol) 1,3-Dibenzyl-5-(benzyloxy)-isophthalat gegeben, zum Rückfluss erwärmt und für 14 Stunden gerührt. Anschließend wird das Reaktionsgemisch mit verdünnter Salzsäure neutralisiert. Es wird mehrfach mit Chloroform extrahiert. Die organischen Phasen werden vereinigt und einige Male mit Wasser gewaschen. Man trocknet über Magnesiumsulfat, filtriert und entfernt das Lösungsmittel im Vakuum. Das verbleibende Öl wird nochmals in Dichlormethan aufgenommen und mit 1 N NaOH ausgeschüttelt. Die vereinigten wässrigen Phasen werden mit Salzsäure angesäuert (pH = 1,5), woraufhin sich ein weißer Feststoff abscheidet. Das Produkt wird abfiltriert und im Hochvakuum getrocknet (2,05 g, 75 %). ¹H-NMR (400 MHz, CDCl₃) δ = 8.22 (s, 1H), 7.78 (s, 2H), 7.45-7.33 (m, 5H), 5.16 (s, 2H).

### Beispiel 8

### Darstellung von 1,3-Diphenyl-5-(benzyloxy)-isophthalat

1,23 g (5 mmol) 1-Benzyloxyisophthalsäure werden in 18,44 g (155 mmol) Thionylchlorid gelöst und unter Argon unter Rückfluss gekocht. Nach Ende der Gasentwicklung wurde der Überschuss an Thionylchlorid im Vakuum abdestilliert. Der verbleibende Rückstand wird in 10 ml Dichlormethan gelöst und langsam zu einer Mischung von 1,13 g (12 mmol) Phenol gelöst in 20 ml Dichlormethan und 6 ml Pyridin (113 mmol) getropft. Das Reaktionsgemisch wird für 24 Stunden bei Raumtemperatur gerührt. Es wird 1× mit 50 ml 1 N NaOH und 3× mit je 50 ml gesättigter NaCl-Lösung extrahiert. Die organische Phase wird über Magnesiumsulfat getrocknet, filtriert und das Lösungsmittel im Vakuum entfernt. Das Produkt wird bei 40°C im Hochvakuum getrocknet (1,95 g, 92 %).
¹H-NMR (400 MHz, CDCl₃) δ = 8.83 (s, 1H), 8.05 (s, 2H), 7.5-7.2 (m, 15H), 5.21 (s, 2H).

### Beispiel 9

### Darstellung von 1,3-Diphenyl-5-hydroxyisophthalat

1,65 g (3,9 mmol) 1,3-Diphenyl-5-(benzyloxy)-isophthalat werden in 50 ml Ethylacetat gelöst. Zu dieser Lösung gibt man unter Stickstoff 0,83 g (0,8 mmol) Pd/C (10 %) und leitet für 4 Stunden Wasserstoff ein. Das Gemisch wird filtriert und das Lösungsmittel im Vakuum entfernt. Man erhält ein weißes Pulver (1,1 g, 85 %). ¹H-NMR (400 MHz, CDCl₃) δ = 10.25-10.15 (s, 1H), 8.33 (s, 1H), 7.83 (s, 2H), 7.45 (m, 4H), 7.31 (m, 2H), 7.25 (m, 4H).

Entsprechend der vorgestellten Beispiele 1 - 5 bzw. 6 - 9 können in ähnlicher Form weitere Phenole erhalten werden:

| Bsp.- Nr.: | Endgruppe | Struktur | Kommentar |
|---|---|---|---|
| 10 | 1,3-Bis-(isoctylphenyl)-5-hydroxyisophthalat | 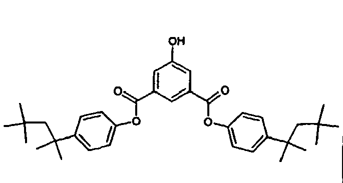 | weißer Feststoff |
| 11 | 1,3-Bis-[3,5-di-(tert.-butyl)-phenyl]-5-hydroxyisophthalat | 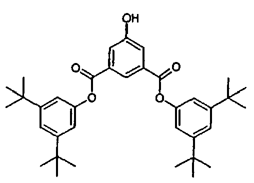 | weißer Feststoff |
| 12 | 1,3-Bis-(p-cumylphenyl)-5-hydroxyisophthalat | 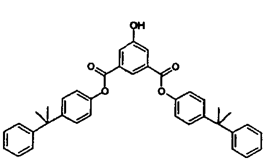 | weißer Feststoff |
| 13 | 1,3-Dicyclodecyl-5-hydroxyisophthalat | 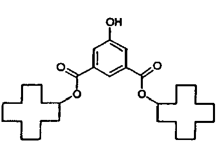 | Öl |
| 14 | 1,3-Dicyclooctyl-5-hydroxyisophthalat | 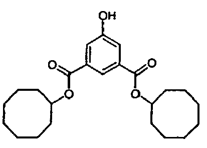 | Öl |
| 15 | 1,3-Bis-[3,5-(diphenyloxycarbonyl)-phenyl]-5-hydroxyisophthalat | 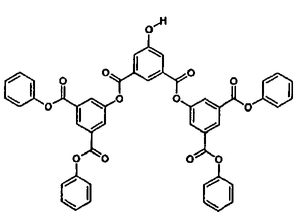 | Weißer Feststoff |

### Beispiel 16

### 3,5-Di-(4-metylbenzoyl)phenol

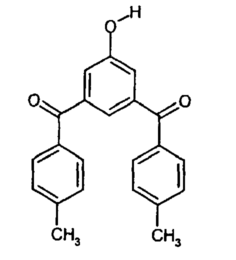

hellgelber Feststoff

### Beispiel 17

### 3,5-Di-(4-tert.butylbenzoyl)phenol

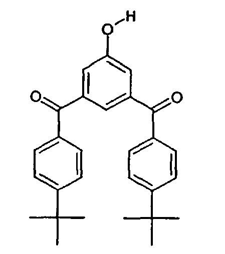

weißer Feststoff

### Beispiel 18

### 3,5-Diphenyloxyphenol

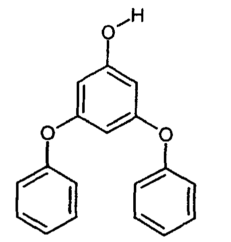

### Beispiel 19

### Darstellung von Polycarbonat mit verzweigter Endgruppe

In einem Kolben werden bei Raumtemperatur 13.698 g (0,06 mol) 2,2-Bis-(4-hydroxyphenyl)-propan und 5.280 g NaOH (220 mol-%, bezogen auf Bisphenol-Komponente) in 220 ml Wasser unter Stickstoffatmosphäre gelöst. Zu diesem Gemisch werden 1.003 g 1,3-Diphenyl-5-hydroxyisophthalat (aus Beispiel 9) gelöst in 220 ml Dichlormethan gegeben. Man lässt für 15 Minuten Rühren und gibt zu diesem Gemisch 0,08 ml (1 mol-%) N-Ethylpiperidin. Bei Raumtemperatur und unter kräftigem Rühren wird 8,3 ml (200 mol-%, bezogen auf Bisphenol-Komponente) Phosgen eingeleitet. Der pH-Wert wird dabei durch Nachdosieren von verdünnter NaOH-Lösung im Bereich von 12,5 - 13 gehalten. Man lässt noch 30 Minuten Rühren. Daraufhin wird mit Dichlormethan verdünnt und die organische Phase abgetrennt. Nach Ansäuren der organischen Phase mit verdünnter Salzsäure wird mit Wasser elektrolytfrei gewaschen. Das in der organischen Phase gelöste Polymer wird in Methanol gefällt.
Ausbeute: 14,8 g
M_{w} = 16240 g/mol
Mₙ = 7830 g/mol
D = 2,07

### Vergleichsbeispiel 20

Die Ausführung des Versuchs entspricht der Vorschrift von Beispiel 16. Als Kettenabbrecher wurde statt 1,3-Diphenyl-5-hydroxyisophthalat *tert.*-Butylphenol eingesetzt.
M_{w} = 16630 g/mol
Mₙ = 7920 g/mol
D = 2,09

| Polycarbonat | Nullviskosität (Pa•s) (bei 270°C) | Molgewicht (g/mol) | Glas-temperatur (°C) |
|---|---|---|---|
| Beispiel 19 | 182 | M_{w} = 16240 | 142 |
| | | Mₙ = 7830 | |
| Vergleichsbeispiel 20 | 216 | M_{w} = 16630 | 145 |
| | | Mₙ = 7920 | |

Anhand vorstehend gezeigter Tabelle wird ersichtlich, dass das erfindungsgemäße Polycarbonat im Vergleich zu einem Polycarbonat mit *tert*.-Butylphenol als Endgruppe bei annähernd identischem Molekulargewicht eine deutlich reduzierte Nullviskosität aufweist.

## Patentansprüche

1. Verwendung von Phenolen der Formel 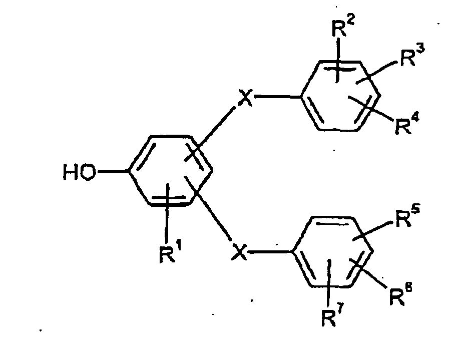 worin
R¹, R², R⁷ unabhängig voneinander für H, lineares oder verzweigtes C₁-C₁₈ Alkyl-, Cl, oder Br stehen,
X eine Einfachbindung oder ein divalentes Radikal ausgewählt aus der Gruppe -CO₂-, -O-, -CH₂- und -CO- sein kann,
R³-R⁶ unabhängig voneinander für H, lineare oder verzweigte C₁-C₁₈ Alkyl-, cyclisches C₅-C₁₈ Alkyl-, Phenyl-, Phenyloxy-, Phenylcarboxy-, Benzyl-, Benzyloxy-, Naphthyl oder Naphthyloxy-, Naphthylcarboxyreste stehen, zur Herstellung endgruppenmodifizierter Polycarbonate und Polyestercarbonate.

2. Polycarbonat oder Polyestercarbonat enthaltend mindestens eine Endgruppe der Formel 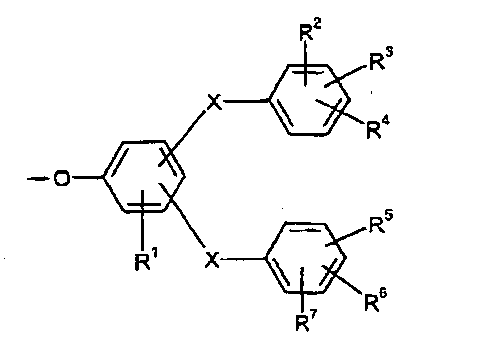 wobei
R¹ - R⁷ und X die in Anspruch 1 angegebene Bedeutung haben.

3. Herstellung der Polycarbonate oder Polyestercarbonate gemäß Anspruch 2, **dadurch gekennzeichnet, dass** Phenole gemäß Anspruch 1 eingesetzt werden.

4. Phenole der Formel 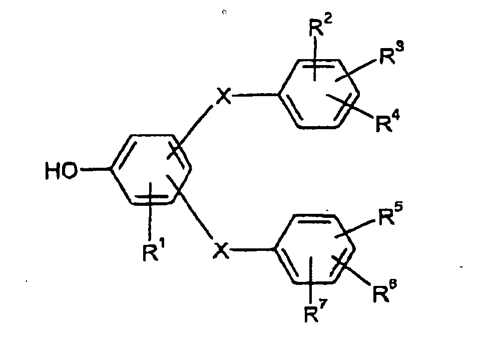 worin
R¹, R², R⁷ unabhängig voneinander für H, lineares oder verzweigtes C₁-C₁₈ Alkyl-, Cl, oder Br stehen,
X eine Einfachbindung oder ein divalentes Radikal wie -CO₂-, -O-, -CH₂- oder -CO- sein kann,
R³-R⁶ unabhängig voneinander für H, lineare oder verzweigte C₁-C₁₈ Alkyl-, cyclisches C₅-C₁₈ Alkyl-, Phenyl-, Benzyl- oder Naphthylreste stehen,
wobei Bis(C₁-C₁₈-alkylphenyl)-4-hydroxyisophthalate, Diphenylhydroxyisophthalat, Bis-[(diphenyloxycarbonyl)-phenyl]-hydroxyisophthalat, Diphenyloxyphenol, 3,5-Dibenzoylphenol, 4-Methyl-2,6-dibenzoylphenol, 2-Metyl-4,6-dibenzoylphenol sowie 3,5-Bis-(4-hydroxybenzoyl)-phenol ausgenommen sind.

5. Verwendung von Polycarbonaten oder Polyestercarbonaten gemäß Anspruch 2 zur Herstellung von Formkörpern und Extrudaten.

6. Formkörper und Extrudate aus Polycarbonaten oder Polyestercarbonaten gemäß Anspruch 2.

## Claims

1. Use of phenols having the formula 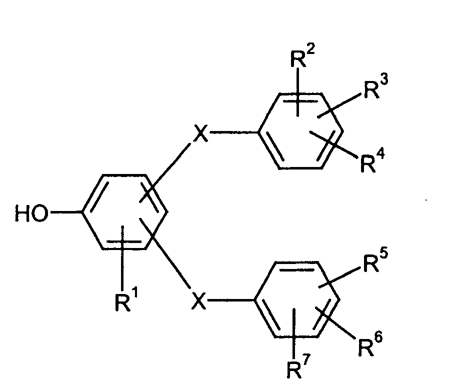 wherein
R¹, R², R⁷ mutually independently stand for H, linear or branched C₁-C₁₈ alkyl, Cl, or Br,
X can be a single bond or a divalent radical selected from the group comprising -CO₂- -O-, -CH₂- and -CO-,
R³-R⁶ mutually independently stand for H, linear or branched C₁-C₁₈ alkyl, cyclic C₅-C₁₈ alkyl, phenyl, phenyloxy, phenylcarboxy, benzyl, benzyloxy, naphthyl or naphthyloxy, naphthylcarboxy radicals, for the production of end group-modified polycarbonates and polyester carbonates.

2. Polycarbonate or polyester carbonate containing at least one end group having the formula 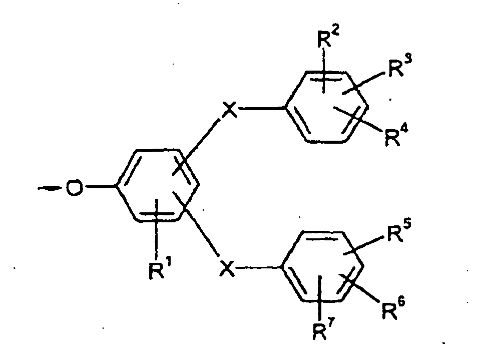 wherein
R¹ to R⁷ and X have the meaning given in claim 1.

3. Production of the polycarbonates or polyester carbonates according to claim 2, **characterised in that** phenols according to claim 1 are used.

4. Phenols having the formula 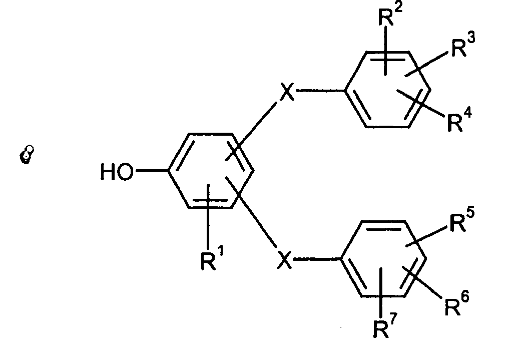 wherein
R¹, R², R⁷ mutually independently stand for H, linear or branched C₁-C₁₈ alkyl, Cl, or Br,
X can be a single bond or a divalent radical such as -CO₂-, -O-, -CH₂- or -CO-,
R³-R⁶ mutually independently stand for H, linear or branched C₁-C₁₈ alkyl, cyclic C₅-C₁₈ alkyl, phenyl, benzyl or naphthyl radicals,
wherein bis(C₁-C₁₈ alkyl phenyl)-4-hydroxyisophthalates, diphenyl hydroxyisophthalate, bis[(diphenyl oxycarbonyl) phenyl] hydroxyisophthalate, diphenyl oxyphenol, 3,5-dibenzoyl phenol, 4-methyl-2,6-dibenzoyl phenol, 2-metyl-4,6-dibenzoyl phenol and 3,5-bis(4-hydroxybenzoyl) phenol are excluded.

5. Use of polycarbonates or polyester carbonates according to claim 2 for the production of moulded parts and extrudates.

6. Moulded parts and extrudates produced from polycarbonates or polyester carbonates according to claim 2.

## Revendications

1. Utilisation de phénols de la formule : 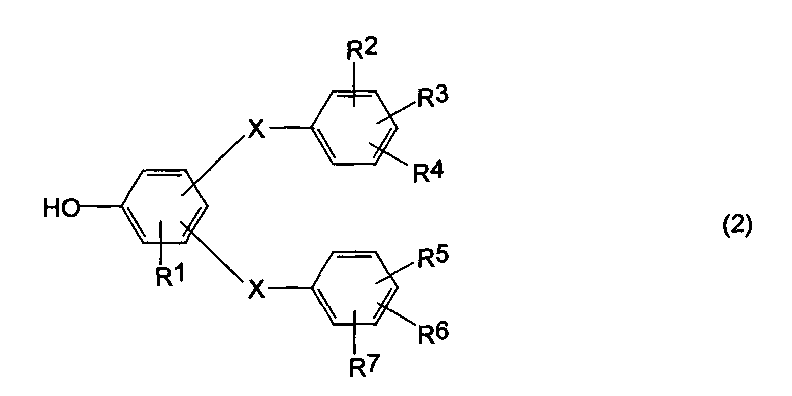 dans laquelle :
R¹, R², R⁷ représentent indépendamment l'un de l'autre, l'atome H, un radical alcoyle en C₁-C₁₈ linéaire ou ramifié, l'atome Cl ou Br,
X peut être une simple liaison ou un radical bivalent parmi le groupe -CO₂-, -O-, -CH₂- ou -CO-,
R³ - R⁶ sont indépendamment l'un de l'autre, l'atome H, un radical alcoyle en C₁-C₁₈ linéaire ou ramifié, alcoyle en C₅-C₁₈ cyclique, phényle, phényloxy, phénylcarboxy, benzyle, benzyloxy, naphtyle ou naphtyloxy, un reste naphtylcarboxy,
pour la préparation de polycarbonates et polyester-carbonates à groupes terminaux modifiés.

2. Polycarbonates ou polyester-carbonates contenant au moins un groupe terminal de la formule : 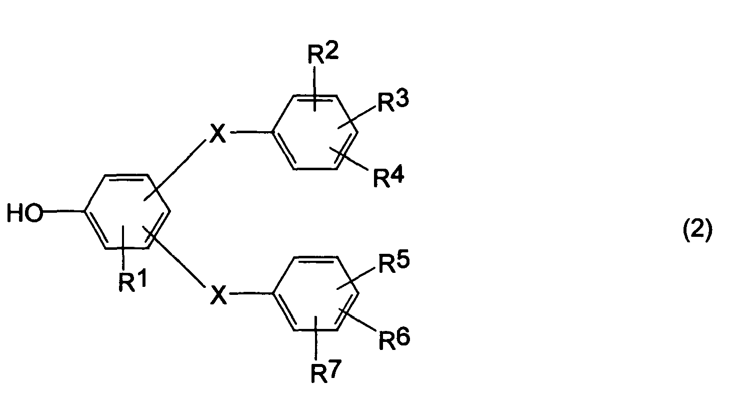 dans laquelle :
R¹ - R⁷ et X ont la signification donnée à la revendication 1.

3. Préparation de polycarbonates et polyester-carbonates selon la revendication 2, **caractérisée en ce que** l'on met en oeuvre des phénols selon la revendication 1.

4. Phénols de la formule 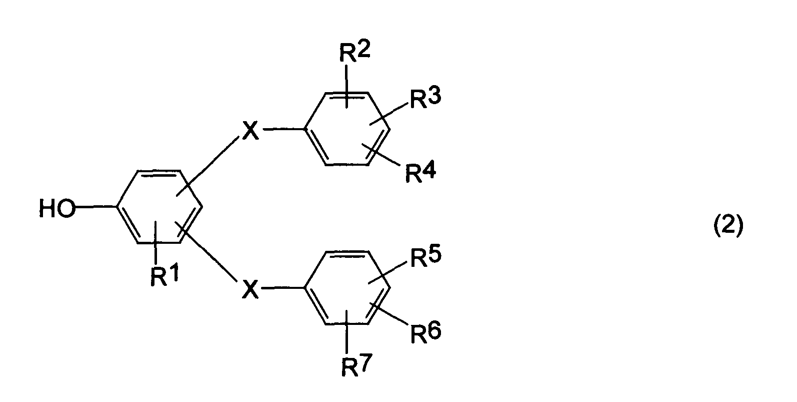 dans laquelle :
R¹, R², R⁷ représentent indépendamment l'un de l'autre, l'atome H, un radical alcoyle en C₁-C₁₈ linéaire ou ramifié, l'atome Cl ou Br,
X peut être une simple liaison ou un radical bivalent comme -CO₂-, -O-, -CH₂- ou -CO-,
R³ - R⁶ sont indépendamment l'un de l'autre, l'atome H, un radical alcoyle en C₁-C₁₈ linéaire ou ramifié, alcoyle en C₅-C₁₈ cyclique, phényle, benzyle, ou naphtyle,
où les 4-hydroxyisophtalates de bis[(alcoyl C₁₋C₁₈)phényle], l'hydroxyisophtalate de diphényle, l'hydroxyisophtalate de bis[(diphényloxycarbonyl)-phényle], le diphényloxyphénol, le 3,5-dibenzoylphénol, le 4-méthyl-2,6-dibenzoylphénol, le 2-méthyl-4,6-dibenzoylphénol, ainsi que le 3,5-bis(4-hydroxybenzoyl)phénol sont exclus.

5. Utilisation de polycarbonates ou polyester-carbonates selon la revendication 2, pour la préparation de corps moulés et d'extrudats.

6. Corps moulés et extrudats en polycarbonates ou polyester-carbonates selon la revendication 2.
